# FASCICULE DE BREVET EUROPEEN

(11) **EP 1 558 306 B1**
(45) Date de publication et mention de la délivrance du brevet: **27.12.2006**
(21) Numéro de dépôt: 03763563.8
(22) Date de dépôt: 08.07.2003
(51) Int. Cl.: A61M 5/30

(54) **DISPOSITIF D INJECTION INTRADERMIQUE ET SOUS CUTANEE**
VORRICHTUNG FÜR DIE INTRADERMALE UND SUBKUTANE INJEKTION
DEVICE FOR INTRADERMAL AND SUBCUTANEOUS INJECTION

(30) Priorité: 11.07.2002 WO PCT/IB02/02703; 28.10.2002 WO PCT/IB02/04494
(43) Date de publication de la demande: 03.08.2005
(73) Titulaire: TecPharma Licensing AG, 3401 Burgdorf (CH)
(72) Inventeur: NERACHER, Arnold, CH-1247 Anières (CH)
(86) Numéro de dépôt international: PCT/CH2003/000455
(87) Numéro de publication internationale: WO 2004/006999

(56) Documents cités:
- GB-A- 681 098
- US-A- 5 911 703
- US-A1- 2002 055 712
- US-B1- 6 258 062

## Description

La présente invention concerne un dispositif d'injection hypodermique de médicaments ou d'autres compositions pharmaceutiques, tels que des hormones de croissance, de l'insuline, de l'héparine et des ADN diverses sous forme liquide. L'invention concerne notamment un dispositif d'injection sans aiguille.

Ce brevet décrit un dispositif permettant de modifier la courbe force/déplacement du ressort entropique décrit dans les demandes de brevets PCT/ IB 02/02703 et PCTAB02/04494.

Dans les demandes ci-dessus la courbe force/déplacement qui dépend du type de matériau élastomère utilisé et de sa structure et qui est une propriété non linéaire du matériau utilisé, peut, dans certains cas d'injection, ne pas être adapté au profil idéal force/déplacement nécessaire.

Le brevet GB 681 098 divulgue un dispositif ayant deux générateurs de force montés en cascade et séparés par une paroi mobile libre.

Au vu des inconvénients mentionnés ci-dessus, le but de la présente invention est de réaliser un dispositif d'injection hypodermique sans aiguille, permettant d'étendre la plage d'utilisation de l'injecteur en modifiant le profil de la courbe force/déplacement. On peut déplacer le point d'inflexion, augmenter le rapport entre la force primaire et la force secondaire, modifier la pente de la courbe de la force primaire.

Les buts de l'invention sont réalisés par le dispositif d'injection hypodermique selon la revendication 1.

L'invention permet de modifier la caractéristique de la courbe force/déplacement par l'utilisation de une ou plusieurs parois mobiles 3, 3a entre lesquelles sont placés des disques en polymère réticulé 10, 10a. Ces disques perforés au centre peuvent avoir des caractéristiques différentes (dureté, alvéoles, diamètre, épaisseur, forme géométrique) permettant de modifier la caractéristique de la courbe force/déplacement donnée par l'utilisation d'un ressort entropique unique.

Chacunes des parois mobiles 3, 3a peut-être munie d'une entretoise 20 de longueur différente permettant de limiter la force d'écrasement du bloc élastique (ressort ou générateur entropique).
De cette façon il est possible d'obtenir un point d'inflexion (I) à une valeur précise de (x) et une pente plus abrupte ce qui permet une transition plus rapide entre la pression primaire et secondaire d'injection permettant de limiter le dégâts dans les tissus par la force du jet initial.

Les parois mobiles libres 3a sont montées en cascade avec ou sans entretoises 20 selon le profil de la courbe force/déplacement que l'on désire, mais on peut également combiner le ou les blocs élastiques (ressort entropique) avec un ressort métallique classique ou un ressort moléculaire (polysiloxane) ou un ballon caoutchouc 30 en forme de torr rempli d'azote 30a

Par exemple un ballon de caoutchouc 30 est pressurisé avec de l'azote, celui-ci génère la force secondaire (basse pression) nécessaire pour transférer le liquide dans le corps. La haute pression est générée par un bloc d'élastomère aplati entre les parois 2a et 3a.

Un autre avantage de la segmentation en cascade des parois mobiles libres 3a permet de créer une double ou triple pressions différentes d'injection par l'utilisation de matériaux présentant des caractéristiques force/déplacement différents.
Par exempte: ressort entropique / ressort métallique; ressort entropique / ressort moléculaire; ressort entropique / ballon de gaz; sans avoir recours à une courbe non linéaire comme dans le cas du ressort entropique seul.

D'autres buts et aspects avantageux de l'invention ressortiront de la description et des revendications ci-après et des dessins annexés, dans lesquels:
- la figure 1: est une coupe longitudinal d'un dispositif d'injection cascade 2 étages en état de charge avant injection
- la figure 2: est une coupe longitudinal d'un dispositif de la figure1 durant la phase de décharge
- la figure 3: est une coupe longitudinal d'un dispositif similaire à la figure1 mais utilisant un ballon en forme de torr pressurisé par de l'azote
- la figure 4: est un diagramme montrant la force générée en fonction du déplacement (x) de la tige de piston 4 pour différentes caractéristiques de ressorts B et C par rapport au ressort entropique simple A.

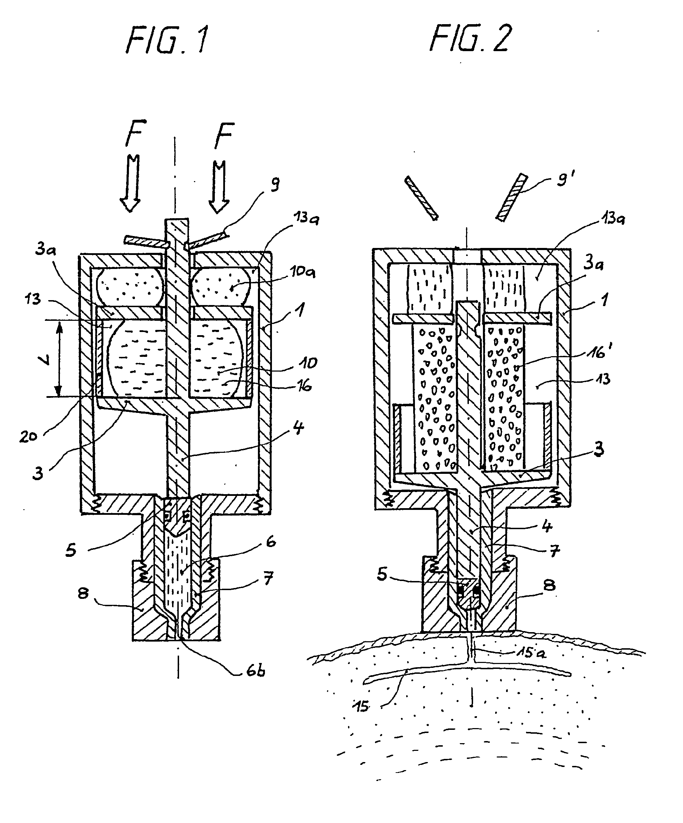

En se référant aux figures 1 à 3, un dispositif d'injection 1 pour l'administration d'un liquide 6 sous la peau 14 d'un patient humain ou d'un animal, comporte une ampoule 7 munie d'un piston 5 faisant partie intégrante de l'ampoule, un élément de transmission 4, un moyen de retenue 9 et des éléments générateurs de forces 10; 10a compris entre deux ou plusieurs parois mobiles libres 3a et une paroi fixe 2a faisant partie intégrante du corps de l'injecteur et une paroi 3 reliée au piston 5 mobile (4, 5). Cette paroi (mobile) 3 est reliée à la tige de piston 4 de manière fixe ou réglable. Si un réglage de course du piston tige 4 est nécessaire, alors on munira ces deux éléments d'un filetage. Les parois mobiles libres 3a sont eux coulissant sur la tige de piston 4. Les parois mobiles 3, 3a peuvent êtres munies d'entretoises 20(butées) qui permettent suivant leurs longueur L de modifier la forme de la courbe et de déplacer le point d'inflexion à une valeur bien précise.

La figure 1 montre l'injecteur en état de charge juste avant l'injection. Cet injecteur est composé un montage en cascade de deux générateurs/ressorts entropiques 10 et 10a présentant des caractéristiques force/déplacement différents. Il se compose du disque de polymère réticulé alvéolaire 10 pour l'élément générant la basse pression et d'un disque de polymère plein 10a pour l'élément créant la pression primaire servant à percer la peau du patient. Chaque disque est percé en son centre par un trou permettant à la tige de piston 4 de coulisser à travers. Le diamètre des disques doit être 20 à 30% plus petit que le diamètre intérieur du corps de l'injecteur 2a ou de l'entretoise 20 de manière que lors de l'aplatissement de ceux-ci, la matière puisse fluer vers l'extérieur sans toucher la paroi extérieure ce qui modifierai considérablement ses caractéristiques. Le ressort entropique ne change pas de volume durant l'aplatissement. Un espace libre 13 et 13a est agencé pour éviter le contact du ressort 10 et 10a avec le boitier 2a ou l'entretoise 20.

La charge de l'injecteur se réalise par le rapprochement des parois mobiles 3, 3a contre la paroi fixe 2a en tirant sur la tige de piston 4 par divers moyens mécaniques (bras de levier, écrou) ou pneumatique (vérin à air). Le déclencheur 9 est arrimé sur la tige de piston 4 de manière à maintenir l'injecteur en état de charge prêt à l'emploi. En applicant une pression F sur le déclencheur 9, celui-ci libère la tige de piston 4 qui va pousser le piston 5 et éjecter le liquide 6 à travers l'orifice 6b.

L'ampoule 7 à usage unique se compose d'un corps 7 en verre ou poly carbonate muni à son extrémité d'une buse transformant l'énergie de pression en énergie cinétique communiquée au micro jet de liquide.

La figure 2 montre l'injecteur après le déclenchement en fin d'injection dans la partie intra dermale 15. Lors du déclenchement de l'injecteur la force générée par le ressort 10a va créer une haute pression dans la capsule 7 nécessaire à percer la peau du patient (canal 15a) puis lorsque la détente est complète alors arrive le relais du ressort 10 qui va générer une faible pression pour permettre au liquide d'être transféré depuis la capsule 7 à la zone à injecter15. Dans le cas d'un ressort cellulaire comme celui dessiné en figure 1 les cellules vides 16 sont complètement aplaties et lors de la détente comme le montre La figure 2, elles reprennent leurs formes originales 16'

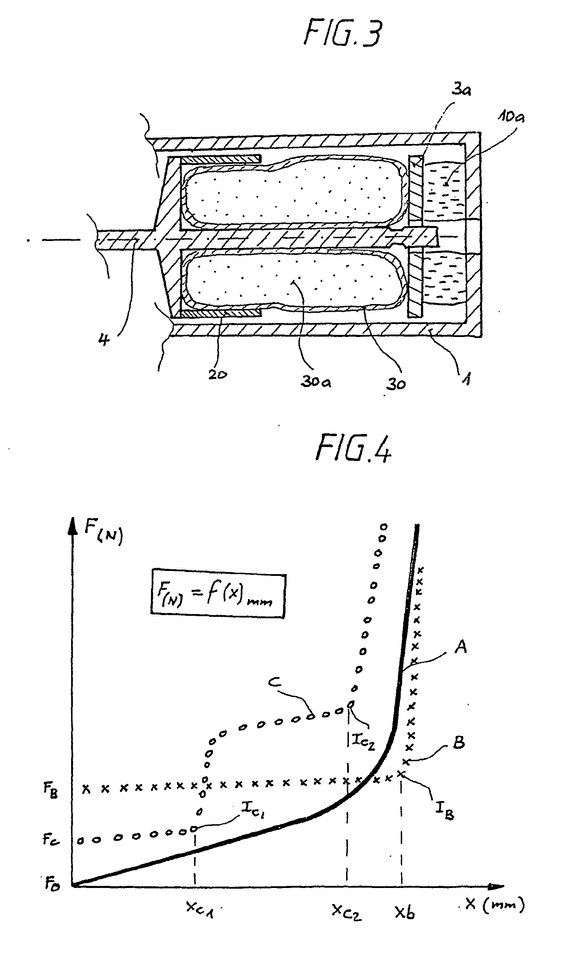

La figure 3 montre une variante de La figure1, ou le ressort 10 est remplacé par un ballon ou un torr en caoutchouc 30 pressurisé par de l'azote. L'azote présente au faible taux de diffusion à travers la membrane caoutchouc ce qui permet de garder la pression sur une longue période sans perte notable de pression. Ce ressort à gaz 30 va générer la force secondaire nécessaire à transférer le liquide de la capsule 7 à la zone 15. La force primaire va être générée par le ressort entropique 10a.

La figure 4 montre un diagramme de la force générée par le ressort entropique en fonction du déplacement de la tige de piston 4. Le diamètre du piston 5 va déterminer la pression en MPa que l'on va créer à l'intérieur du récipient 7 durant l'injection. La courbe A est la forme caractéristique d'un ressort entropique.comme décrit dans les demandes de brevet PCT/ IB 02/02703 et PCT/IB02/04494. La courbe B est la forme caractéristique que génère deux ressorts montés en cascade et muni d'une entretoise 20 comme décrit dans ce brevet. On peut constater que le point d'inflexion (I) est très marqué ce qui n'est pas le cas dans la courbe A. L'avantage majeur est que la profondeur d'injection est très précise et est indépendante de la dureté de la peau. Autre différence notable est la valeur de la force générée en fin d'injection F2 due à une précontrainte du ressort 10. Le point d'inflexion (I) en Xb peut-être déplacé sur l'abscisse (x) par la modification de la longueur (L) de l'entretoise 20. La courbe C montre un montage en cascade de trois ressorts aux caractéristiques différentes. Ce montage comprend deux parois mobiles libres 3a et 3b, et d'une paroi mobile 3 arrimée sur la tige de piston 4 munie d'une entretoise 20. Cet agencement permet de générer trois pressions d'injections différentes durant la décharge de l'injecteur.

Les figures 1 et 2 montre un injecteur dont le déclencheur se trouve situé derrière le ressort entropique. On peut également réaliser le même injecteur avec le déclencheur situé entre l'ampoule et le ressort entropique.

Les possibilités d'agencement des ressorts de diverses nature, le nombre de parois mobiles libres, la forme des disques de polymère réticulé ne se limite pas seulement à celles décrite dans le brevet.

## Revendications

1. Dispositif d'injection hypodermique (1) pour l'administration sans aiguille d'un produit liquide, comportant un réservoir (7) contenant ledit produit liquide à injecter (6) entre un piston mobile (5) et un orifice (6b) et au moins deux générateurs de force (10,10a) montés en cascade, dont le premier générateur est situé entre l'arrière intérieur du corps (1) et une paroi mobile libre (3a) et le deuxième générateur est situé entre ladite paroi mobile libre (3a) et une paroi (3) reliée au piston (4, 5), **caractérisé en ce que** la paroi (3) reliée au piston (4, 5) ou la /les paroi(s) mobiles libres (3a) sont munies d'entretoises amovibles (20) qui peuvent être de longueur (L) différente.

2. Dispositif selon la revendication 1, **caractérisé en ce que** le piston (5) est prolongé par une tige (4).

3. Dispositif selon la revendication 1, **caractérisé en ce que** la paroi mobile libre (3a) coulisse sur la tige (4) du piston (5).

4. Dispositif selon la revendication 1, 2 ou 3, **caractérisé en ce que** plusieurs générateurs (10a) sont situés entre plusieurs parois mobiles libres (3a).

5. Dispositif selon l'une des revendications 1 à 4, **caractérisé en ce que** les générateurs (10, 10a) ayant des caractéristiques diverses permettent de créer une force en fonction du déplacement qui permette une injection précise en quantité comme en profondeur.

6. Dispositif selon l'une des revendications 1 à 5, **caractérisé en ce que** les générateurs (10, 10a) peuvent être de nature diverses tels que; ressort entropique; ressort moléculaire; ressort acier; ressort magnétique ou gaz sous pression.

7. Dispositif selon la revendication 6, **caractérisé par le fait que** les générateurs (10,10a) de nature diverses peuvent êtres combinés entre eux.

8. Dispositif selon la revendication 1, **caractérisé par** l'injection hypodermique à usage unique ou multi-usage pour l'administration intra dermal ou sous cutané ayant des moyens de rétention permettant de maintenir, avant l'utilisation, une disque à une force suffisant pour créer dans l'ampoule une pression pour propulser le produit liquide à injecter, pendant l'utilisation, à travers l'orifice de manière à créer un jet de liquide ayant une vitesse suffisante pour traverser la peau du patient ou de l'injecter à la bonne profondeur.

## Claims

1. Hypodermic injection device (1) for the needle-free administration of a liquid product, comprising a reservoir (7) containing said liquid product to be injected (6) between a movable piston (5) and an orifice (6b) and at least two force generators (10, 10a) mounted in cascade, of which the first generator is located between the inner rear of the body (1) and a free movable wall (3a) and the second generator is located between said free movable wall (3a) and a wall (3) connected to the piston (4, 5), **characterised in that** the wall (3) connected to the piston (4, 5) or the free movable wall(s) (3a) are equipped with removable struts (20) which may differ in length (L).

2. Device according to claim 1, **characterised in that** the piston (5) is extended by a rod (4).

3. Device according to claim 1, **characterised in that** the free movable wall (3a) slides over the rod (4) of the piston (5).

4. Device according to claim 1, claim 2 or claim 3, **characterised in that** a plurality of generators (10a) are located between a plurality of free movable walls (3a).

5. Device according to any one of claims 1 to 4, **characterised in that** the generators (10, 10a) having various characteristics produce a force as a function of the displacement which allows precise injection both in terms of quantity and in terms of depth.

6. Device according to any one of claims 1 to 5, **characterised in that** the generators (10, 10a) may be of various types such as: an entropic spring; a molecular spring; a steel spring; a magnetic spring or a compressed gas.

7. Device according to claim 6, **characterised by** the fact that the generators (10, 10a) of various types may be mutually combined.

8. Device according to claim 1, **characterised by** single-use or multiple-use hypodermic injection for intradermal or subcutaneous administration, having retention means allowing a disc to be held, prior to use, at a sufficient force to produce in the ampoule a pressure for propelling the liquid product to be injected, during use, through the orifice in such a way as to produce a jet of liquid having a sufficient speed to pass through the patient's skin or to inject said liquid at a suitable depth.

## Patentansprüche

1. Vorrichtung (1) zur hypodermalen Injektion für die nadellose Verabreichung eines flüssigen Produkts, die ein Reservoir (7), das das zu injizierende flüssige Produkt (6) zwischen einem beweglichen Kolben (5) und einer Öffnung (6b) enthält, und mindestens zwei kaskadierte Krafterzeugungsmittel (10, 10a) aufweist, wobei das erste Erzeugungsmittel zwischen dem inneren Hinterende des Körpers (1) und einer freien beweglichen Wand (3a) und das zweite Erzeugungsmittel zwischen der freien beweglichen Wand (3a) und einer mit dem Kolben (4, 5) verbundenen Wand (3) angeordnet sind, **dadurch gekennzeichnet, dass** die mit dem Kolben (4, 5) verbundene Wand (3) oder die freie/n bewegliche/n Wand/Wände (3a) mit beweglichen Abstandshaltern (20) versehen sind, die eine unterschiedliche Länge (L) haben können.

2. Vorrichtung nach Anspruch 1, **dadurch gekennzeichnet, dass** der Kolben (5) durch eine Stange (4) verlängert ist.

3. Vorrichtung nach Anspruch 1, **dadurch gekennzeichnet, dass** die freie bewegliche Wand (3a) auf der Stange (4) des Kolbens (5) gleitet.

4. Vorrichtung nach Anspruch 1, 2 oder 3, **dadurch gekennzeichnet, dass** mehrere Erzeugungsmittel (10a) zwischen mehreren freien beweglichen Wänden (3a) angeordnet sind.

5. Vorrichtung nach einem der Ansprüche 1 bis 4, **dadurch gekennzeichnet, dass** dank der Erzeugungsmittel (10, 10a), die über verschiedene Eigenschaften verfügen, eine Kraft in Abhängigkeit von der Verschiebung erzeugt werden kann, die eine präzise Injektion, sowohl was die Menge als auch die Tiefe angeht, gestattet.

6. Vorrichtung nach einem der Ansprüche 1 bis 5, **dadurch gekennzeichnet, dass** die Erzeugungsmittel (10, 10a) unterschiedlicher Art sein können, wie beispielsweise eine entropische Feder, eine molekulare Feder, eine Stahlfeder, eine Magnetfeder oder ein Druckgas.

7. Vorrichtung nach Anspruch 6, **dadurch gekennzeichnet, dass** Erzeugungsmittel (10, 10a) unterschiedlicher Art miteinander kombiniert werden können.

8. Vorrichtung nach Anspruch 1, **gekennzeichnet durch** die einmalige oder mehrmalige hypodermale Injektion für die intradermale oder subkutane Verabreichung mit Haltemitteln, dank derer vor dem Gebrauch eine Scheibe mit einer solchen Kraft gehalten werden kann, dass in der Ampulle ein Druck zum Antrieb des zu injizierenden flüssigen Produkts erzeugt wird, und während des Gebrauchs über der Öffnung, um einen Flüssigkeitsstrahl mit einer solchen Geschwindigkeit zu erzeugen, dass er die Haut des Patienten durchquert oder hinreichend tief injiziert wird.
